# EUROPEAN PATENT APPLICATION

(11) **EP 3 431 014 A1**
(43) Date of publication of application: **23.01.2019**
(21) Application number: 17766429.9
(22) Date of filing: 06.03.2017
(51) Int. Cl.: A61B 17/06

(54) **SUTURE NEEDLE**

(30) Priority: 17.03.2016 JP 2016054401
(71) Applicant: Yoshimi Inc., Obu-shi, Aichi 474-0038 (JP); Akiyama Medical Co., Ltd., Tokyo 113-0033 (JP); Kyushu University, National University Corporation, Fukuoka-shi Fukuoka 812-8581 (JP); Sakuma Special Steel Co., Ltd., Nagoya-shi, Aichi 458-8510 (JP)
(72) Inventor: YOSHIMI, Yukiharu, Obu-shi Aichi 474-0038 (JP); KAWAI, Hirohisa, Tokyo 113-0033 (JP); IKEDA, Tetsuo, Fukuoka-shi Fukuoka 812-8581 (JP); TAKAMATSU, Kazusi, Nagoya-shi Aichi 458-8510 (JP)
(74) Representative: Patentanwälte Bals & Vogel
(86) International application number: PCT/JP2017/008760
(87) International publication number: WO 2017/159427

(57) **Abstract**

A suture needle for endoscopic surgery, may include: a needle body; a suture thread; and a connector configured to connect the needle body and the suture thread, wherein the needle body mat be constituted of a shape memory alloy, may have an arc shape as its original shape, and may be configured to be restored to the arc shape at a predetermined temperature or higher, and the connector may be constituted of a material different from the shape memory alloy.

## Description

### Cross-Reference to Related Application

This application claims priority to Japanese Patent Application No. 2016-054401, filed on March 17, 2016, the entire contents of which are incorporated herein by reference.

### Technical Field

The technique disclosed herein relates to a suture needle for an endoscopic surgery. It is more specifically a suture needle for an endoscopic surgery to which a suture thread is connected.

### Background Art

A minimally invasive surgery has been long desired in surgical procedures in order to lessen traumatic insult on patients. Due to this, in recent surgical procedures, an endoscopic surgery is broadly practiced. The endoscopic surgery does not create a large incision on a patient's body, but it rather creates a small incision on the patient's body and inserts an endoscope or another surgical instrument through the created small incision into the patient's body to perform surgical operations therein (for example, see Patent Literature 1).

### Citation List

### Patent Literatures

Patent Literature 1: Japanese Patent Application Publication No. 2011-224300
Patent Literature 2: Japanese Utility Model Application Publication No. S59-119835

### Summary of Invention

### Technical Problem

In an endoscopic surgery, a surgical instrument is inserted into a patient's body. For example, a suture needle may be inserted to suture an organ or tissue in the patient's body. When the suture needle is inserted, it needs to be inserted through a small incision created on the patient's body, thus a shape of the suture needle is restricted by a size of the incision created on the patient's body. Due to this, use of a suture needle constituted of a shape memory alloy is being considered. This suture needle is constituted of a shape memory alloy, having an arc shape as its original shape, and is configured to be restored to the arc shape at a predetermined temperature or higher. A suture thread is connected to the suture needle.

Shape memory alloy is subjected to a shape-memory thermal treatment, and the shape memory alloy is thereby endowed with a shape memory characteristic that allows it to deform relatively freely at a predetermined temperature (that is, a transformation point) or lower to take a desired shape, as well as allows it to be restored to its originally "memorized" shape at the predetermined temperature (the transformation point) or higher. Further, generally, a temperature in a patient's body is higher than a temperature outside the body. Due to this, by setting the transformation point of the shape memory alloy to be the transformation point (such as 35°C) or higher inside the body and to be the transformation point (such as 35°C) or lower outside of the body, the suture needle can be restored to its original shape when it is inserted into the body. Thus, according to the above suture needle, it becomes possible to deform the suture needle into a desired shape outside the body since the outside of the patient's body is at the predetermined temperature or lower. For example, the suture needle may be cooled to reduce its temperature using a cooling device outside the patient's body, and the cooled suture needle may be deformed by a martensitic transformation. Due to this, the suture needle can be deformed outside the patient's body to a shape that can easily be inserted into the body before inserted into the patient's body. For example, the suture needle can be given a shape that can easily be inserted into the patient's body by being deformed into a ring shape or a linear shape. Due to this, a relatively large suture needle can be inserted into the patient's body safely and fast. Further, according to the above suture needle, since the inside of the patient's body is at the predetermined temperature or higher, the suture needle constituted of shape memory alloy experiences reverse transformation when its temperature rises upon being inserted into the patient's body, and is restored to its original arc shape. Due to this, its needle body can be restored to the arc shape in the patient's body, and a suture procedure can be performed with the original arc-shaped suture needle. As above, according to the above suture needle, since the suture needle is capable of deforming outside the patient's body upon a surgery and being restored to the original arc shape inside the patient's body, the relatively large suture needle can be inserted into the patient's body safely and fast and a surgical operation can thereby be performed.

However, in the above suture needle, there was a problem that the suture needle and the suture thread cannot be firmly connected since the suture needle is constituted of the shape memory alloy. That is, even if the suture needle and the suture thread are connected by a mechanical method (such as crimp fixation), a strong connecting force is difficult to be achieved due to characteristics of the shape memory alloy. Further, the above suture needle is restored to the original shape inside the body after having been deformed outside the patient's body. Due to this, when the suture needle reverse-transforms and is restored to the original shape, phase transformation may occur at a portion where the suture needle and the suture thread are connected and shape dimensions may thereby be changed, which may result in reduction in the connecting force. Further, since a conventional suture needle is constituted of austenitic stainless steel, its connected portion of the suture needle and a suture thread can be processed by puncturing a hole at a center of a rear end portion of the needle, inserting the suture thread therein, and crimping the portion from an outer side, however, since the shape memory alloy has a large elastic force, spring-back would occur despite the crimping and the suture thread cannot be held with a sufficient force. Further, thermal treatment should be performed so that the suture thread can be held at the crimped portion, however, the suture thread cannot withstand heat in the thermal treatment.

Meanwhile, a suture needle used for a general surgery is described in Patent Literature 2. The suture needle of Patent Literature 2 includes a needle body, a suture thread, and a connector configured to connect the needle body and the suture thread. The needle body includes a base end and a projection projecting from the base end. The connector includes an insert hole. The projection of the needle body is inserted to one end portion of the insert hole, and the suture thread is inserted to another end portion of the insert hole. In the suture needle of Patent Literature 2, the connector is constituted of a shape memory alloy, and the needle body is constituted of metal that is different from the shape memory alloy. When the needle body and the suture thread are to be connected by the connector, the connector is set to be at a transformation point temperature of the shape memory alloy or lower, and the projection of the needle body and the suture thread are inserted to the insert hole in a state where an inner diameter of the insert hole of the connector is enlarged. After this, the connector is brought to be at the transformation point temperature of the shape memory alloy or higher to reduce the inner diameter of the insert hole. Due to this, the needle body and the suture thread are tightened by the shape memory alloy connector and are thereby connected.

The technique of Patent Literature 2 is a technique that uses the characteristic of the shape memory alloy in the process of connecting the needle body and the suture thread by the connector, that is, in a process of manufacturing the suture needle. Thus, Patent Literature 2 does not describe a technique that uses the characteristic of the shape memory alloy in a process of using the suture thread in the patient's body by inserting it therein.

Further, the needle body is constituted of the metal that is different from the shape memory alloy in the suture needle of Patent Literature 2, so it cannot be deformed and inserted into the patient's body as the aforementioned shape memory alloy suture needle.

Further, if the connector of Patent Literature 2 is adapted to the suture needle constituted of the shape memory alloy, there is a possibility that the shape memory alloy connector may deform simultaneously as the shape memory alloy suture needle is deformed by being cooled to the transformation point temperature or lower. Due to this, the inner diameter of the insert hole of the connector would be enlarged when the suture needle is deformed, which may result in separation of the needle and the thread from the connector. As above, use of the shape memory alloy in the portion connecting the needle and the thread causes a problem.

Thus, the description herein provides a technique that enables a needle body of a suture needle and a suture thread to be firmly connected.

### Solution to Technical Problem

A suture needle disclosed in this description may be used for endoscopic surgery. This suture needle may comprise a needle body, a suture thread, and a connector configured to connect the needle body and the suture thread. The needle body may be constituted of a shape memory alloy, may have an arc shape as its original shape, and may be configured to be restored to the arc shape at a predetermined temperature or higher. The connector may be constituted of a material different from the shape memory alloy.

According to the above configuration, since the connector is constituted of the material different from the shape memory alloy, a connecting force between the needle body and the suture thread can be increased, and further, a shape of the connector does not change even when a shape of the needle body changes, and thus a decrease in the connecting force between the needle body and the suture thread can be suppressed. Due to this, the needle body and the suture thread of the suture needle can be connected firmly. As above, by using the material different from the shape memory alloy as a material of the connector, the problem caused by using the shape memory alloy at a connecting portion of the needle and the thread is resolved.

### Brief Description of Drawings

FIG. 1 is a side view of a suture needle;
FIG. 2 is a cross-sectional view of a connector.;
FIG. 3 is a cross-sectional view of a primary portion III of FIG. 1;
FIG. 4 is a side view of gripping forceps;
FIG. 5 is a cross-sectional view of a primary portion V of FIG. 4;
FIG. 6 is a diagram (1) explaining a method of using the suture needle;
FIG. 7 is a diagram (2) explaining the method of using the suture needle (and is an enlarged view of a primary portion VII of FIG. 6);
FIG. 8 is a diagram (3) explaining the method of using the suture needle (and is a diagram showing a state where the suture needle is inserted to a guide tube);
FIG. 9 is a diagram (4) explaining the method of using the suture needle (and is an enlarged view of the primary portion VII of FIG. 6 in another state);
FIG. 10 is a diagram explaining a method of using a suture needle of another embodiment; and
FIG. 11 is a diagram explaining a method of using a suture needle of another embodiment.

### Description of Embodiments

Some of primary features characteristic to below-described embodiments will herein be listed. It should be noted that the respective technical elements are independent of one another, and are useful solely or in combinations.

(Feature 1) The needle body may comprise a base end and a projection projecting from the base end. An insert hole may be provided in the connector. The projection may be inserted in one end portion of the insert hole and the suture thread may be inserted in another end portion of the insert hole.

According to such a configuration, the needle body and the suture thread can be connected in a state where the projection of the needle body and an end of the suture thread are inserted in the insert hole of the connector, so they can be connected firmly.

(Feature 2) An end portion of the projection on a base end side of the needle body may be tapered, and one end portion of the connector may be chamfered. The tapered end portion and the chamfered one end portion may face each other.

According to such a configuration, stress at a portion where the needle body and the connector make contact is dispersed, so the needle body becomes resistant to breaking.

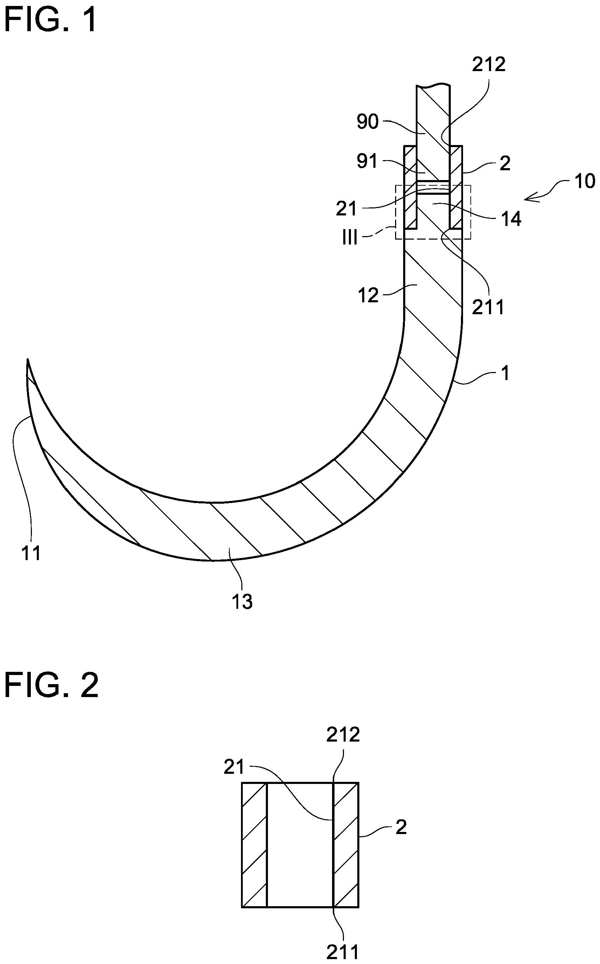

Hereinbelow, embodiments will be described with reference to the attached drawings. As shown in FIG. 1, a suture needle 10 according to an embodiment includes a needle body 1 and a connector 2. A suture thread 90 is connected to the suture needle 10.

The needle body 1 has an arc shape as a whole when it is at a predetermined temperature (transformation point) or higher. The needle body 1 includes a distal end 11, a trunk 13, a base end 12, and a projection 14. The distal end 11 is sharply pointed by being shaped to become narrower towards its end. The trunk 13 has an arc shape and is curved. The base end 12 is adjacent to the connector 2. The projection 14 projects from the base end 12.

The needle body 1 is constituted of shape memory alloy. As the shape memory alloy, for example, Ni-Ti alloy, Cu-Zn-Al alloy, and Ni-Ti-Cu alloy may be exemplified. The shape memory alloy has a shape memory effect or super elasticity, and its shape changes by phase transformation or reverse transformation between an austenite phase and a martensite phase. The needle body 1 constituted of the shape memory alloy has the arc shape as its original shape. Due to the characteristic of the shape memory alloy, when the needle body 1 reaches a predetermined temperature or lower (martensite transformation point temperature Ms point or lower), its Young's modulus starts to decrease, and it becomes deformable when reaching a temperature of a Mf point (martensite transformation terminating temperature) or lower. On the other hand, the needle body 1 starts to be restored from its deformed shape to the originally "memorized" arc shape when reaching a predetermined temperature or higher (austenite transformation point temperature As point or higher), and the Young's modulus increases at an Af point (austenite transformation terminating temperature) or higher and the needle body 1 thus returns to its original shape and enters a super-elastic state. For example, in a case of using Ni-Ti alloy as a material of the needle body 1, values of the respective transformation points (predetermined temperatures) can be adjusted by adjusting a ratio of Ni and Ti and by memory thermal treatment temperature. The predetermined temperature may be set by taking a human body temperature into consideration. For example, the predetermined temperature may be set in a range of 30 to 40°C, and the predetermined temperature of the present embodiment is 35°C (i.e., the body temperature). Further, in general, the temperature in a patient's body is higher than a temperature outside the body, and the predetermined temperature (e.g., 35°C) or higher is achieved inside the body, and the outside of the body is at the predetermined temperature or lower. Due to this, the needle body 1 is at the predetermined temperature or lower when it is outside the patient's body, and thus is deformable. On the other hand, the needle body 1 is at the predetermined temperature or higher when it is within the patient's body, and thus is restored to the original arc shape. For example, in a case where the predetermined temperature is 35°C, when the needle body 1 is in an environment with 35°C or lower outside the patient's body, its Young's modulus decreases and thus it is easily deformed or becomes freely deformable. On the other hand, when the needle body 1 is introduced into the patient's body having the body temperature of 35°C or higher, it experiences reverse transformation and is restored to the original arc shape. The patient's body can be sutured by the needle body 1 returning to its arc shape.

As shown in FIG. 2, the connector 2 has a cylindrical shape. The connector 2 includes an insert hole 21. The insert hole 21 may be formed, for example, by laser processing or drilling. The insert hole 21 is provided to extend in an axial direction of the connector 2. As shown in FIG. 1, an outer diameter of the connector 2 is preferably the same as an outer diameter of the base end 12 of the needle body 1. The projection 14 of the needle body 1 is inserted to a distal end portion 211 of the insert hole 21 of the connector 2. A distal end 91 of the suture thread 90 is inserted to a proximal end portion 212 of the insert hole 21. The needle body 1 and the suture thread 90 are connected by the connector 2 being pressured from its periphery in a state where the projection 14 of the needle body 1 and the distal end 91 of the suture thread 90 are inserted in the insert hole 21. That is, the needle body 1, the suture thread 90, and the connector 2 are fixed by crimping. The connector 2 connects the needle body 1 and the suture thread 90 by the crimping fixation.

The connector 2 is constituted of a material different from that of the needle body 1. That is, the connector 2 is constituted of a material that is different from the shape memory alloy. The connector 2 is constituted of austenitic stainless steel, for example. As the material of the connector 2, austenitic stainless steel used as a material of a suture needle in general is preferable. As such austenitic stainless steel, for example, SUS304, SUS316, SUS316L, and SUS317 may be exemplified. The connector 2 does not have the characteristic of the shape memory alloy as the needle body 1 does. Thus, the connector 2 does not deform or return to its original shape by the predetermined temperature. The material of the connector 2 is not limited to austenitic stainless steel. The connector 2 may be constituted of composite resin, for example.

Oxide films are provided on surfaces of the needle body 1 and the connector 2. The oxide films may be formed by heat-treating the needle body 1 and the connector 2. The oxide films suppress glossiness of the needle body 1 and the connector 2. Further, the glossiness may be suppressed by removing the oxide films formed on the surfaces of the needle body 1 and the connector 2 by pickling or polishing, and then sandblasting the surfaces. This process is performed for a purpose of reducing eye fatigue caused by reflected light from the needle, because a surgeon performs surgery using a microscope in precision surgeries.

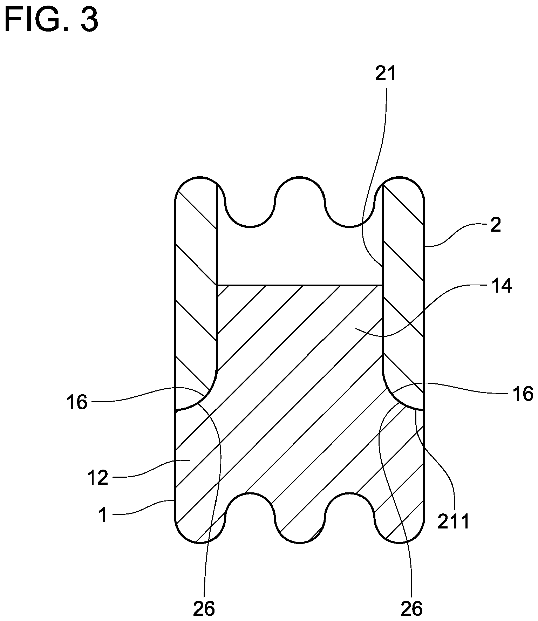

As shown in FIG. 3, an end portion of the projection 14 of the needle body 1 on a base end 12 side has a rounded shape (R), and a tapered portion 16 of which diameter gradually increases toward the base end 12 side is provided. The end portion of the projection 14 of the needle body 1 on the base end 12 side is tapered. The distal end portion 211 of the connector 2 is chamfered, and a chamfered portion 26 is provided. An inner circumferential portion of the connector 2 is chamfered. The tapered portion 16 of the needle body 1 and the chamfered portion 26 of the connector 2 face each other. Upon chamfering, the chamfering is performed so that a corner portion includes a combination of a rounded surface and an angled surface. By so doing, breakage at a step portion of the needle body 1 is reduced, and a gap between the needle body 1 and the connector 2 is decreased as much as possible.

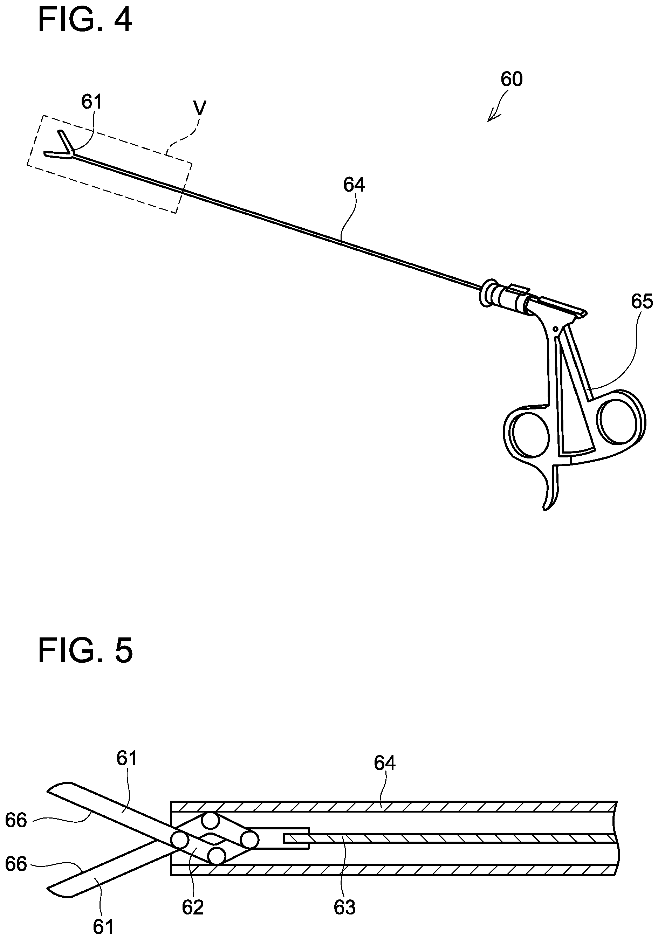

Next, an example of gripping forceps for holding the suture needle will be described. As shown in FIGS. 4 and 5, the gripping forceps 60 include a pair of grip portions 61, a link mechanism 62 coupled to the pair of grip portions 61, and a rod 63 coupled to the link mechanism 62. Further, the gripping forceps 60 include a tube 64 covering the rod 63 and a handle 65 coupled to the rod 63.

The pair of grip portions 61 is provided at a distal end portion of the gripping forceps 60. The pair of grip portions 61 faces each other and is configured to be capable of opening and closing. The pair of grip portions 61 opens and closes in a state where their grip surfaces 66 face each other. When the pair of grip portions 61 is closed, the suture needle 10 is held between the pair of grip portions 61.

The link mechanism 62 is provided between the pair of grip portions 61 and the rod 63. The link mechanism 62 connects the grip portions 61 and the rod 63. The link mechanism 62 transmits motion of the rod 63 to the pair of grip portions 61. The link mechanism 62 converts translational motion of the rod 63 to rotary motion of the grip portions 61. The pair of grip portions 61 opens and closes by rotating via the link mechanism 62.

The rod 63 is provided between the link mechanism 62 and the handle 65. The rod 63 connects the link mechanism 62 and the handle 65. The rod 63 is translated in an axial direction by operation of the handle 65. The rod 63 is arranged inside the tube 64. The tube 64 extends along the rod 63.

The handle 65 is provided at a proximal end portion of the gripping forceps 60. When the handle 65 is operated, the rod 63 is translated. Due to this, the pair of grip portions 61 opens and closes.

Next, a method of using the suture needle provided with the above configuration will be described. As an example, a method of using the suture needle 10 in a laparoscopic surgery will be described. The laparoscopic surgery is a known surgery method, and is a surgery performed on an abdomen of a patient by creating a small incision in the abdomen of the patient instead of radically opening the abdomen of the patient, and is performed by inserting an endoscopy and another surgical instrument to an abdominal cavity of the patient.

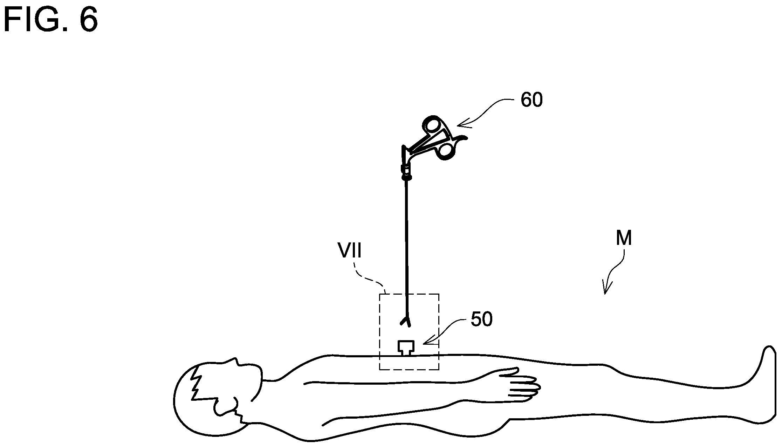

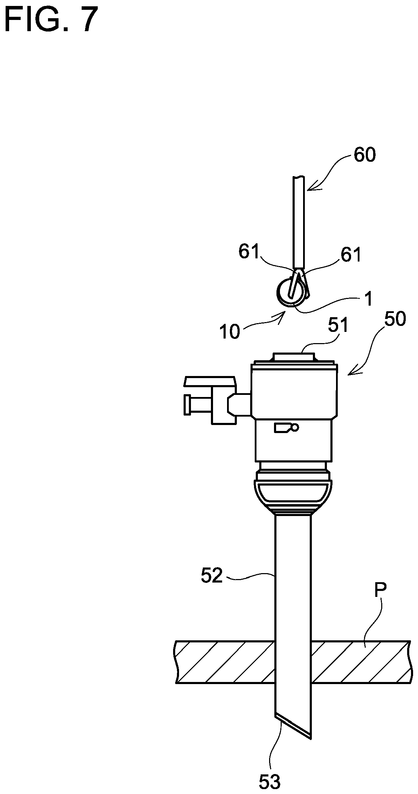

Upon performing the laparoscopic surgery, as shown in FIG. 6, a trocar 50 is inserted to an abdomen of a patient M. The trocar 50 is inserted to an incision created on the abdomen of the patient M. The trocar 50 is a known surgical instrument, and is an instrument for communicating inside and outside of the abdomen of the patient (inside and outside of the body) via the incision created on the abdomen of the patient M upon inserting the endoscopy and other surgical instrument to the abdominal cavity of the patient M. As shown in FIG. 7, the trocar 50 includes an inlet 51, a guide tube 52, and an outlet 53. When the trocar 50 is installed in the abdomen P of the patient, the inlet 51 is open at the outside of the abdomen P of the patient (outside of the body), and the outlet 53 is open at the inside of the abdomen P of the patient (inside of the body). The inlet 51 and the outlet 53 of the trocar 50 communicate with inside of the guide tube 52. The guide tube 52 extends from the outside to the inside of the abdomen P of the patient (from the outside to the inside of the body).

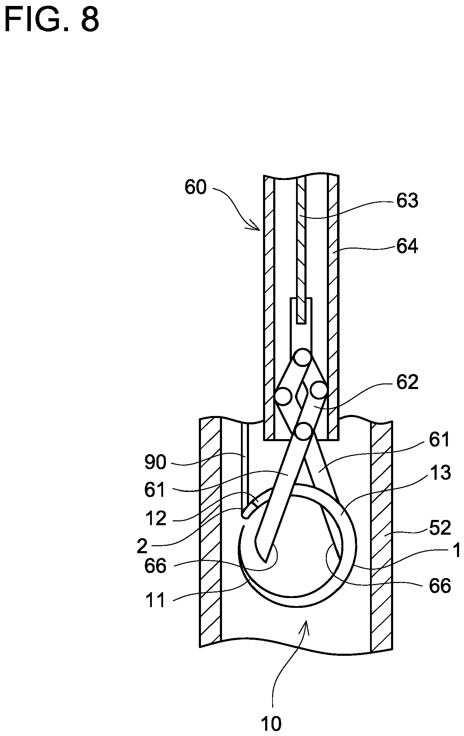

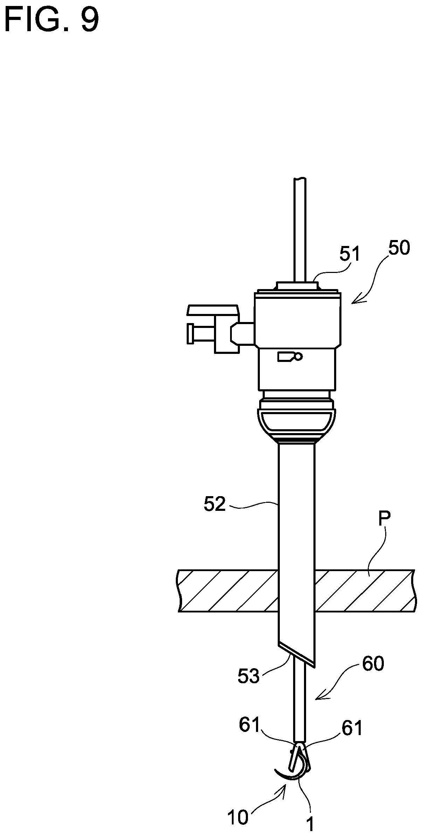

When the suture needle 10 is used, the shape of the arc-shaped needle body 1 of the suture needle 10 is changed outside the abdomen P of the patient (at the outside of the body). Since the outside of the abdomen P of the patient (outside of the body) is at the predetermined temperature or lower, the needle body 1 can be deformed. For example, the needle body 1 is cooled to reduce its temperature by using a cooling device outside the patient's body, and the cooled needle body 1 is deformed. In the example shown in FIG. 7, the arc-shaped needle body 1 is deformed to a ring shape. When the needle body 1 is deformed to the ring shape, the distal end 11 and the base end 12 approach each other. Next, the suture needle 10 is held by the gripping forceps 60 in the state where the needle body 1 has been deformed to the ring shape, and this suture needle 10 is inserted to the inside of the guide tube 52 from the inlet 51 of the trocar 50. As shown in FIG. 8, as the gripping forceps 60 and the suture needle 10 are brought deeper into the guide tube 52, the gripping forceps 60 and the suture needle 10 are guided to the outlet 53 along the guide tube 52. Then, as shown in FIG. 9, the gripping forceps 60 and the suture needle 10 are delivered into the abdominal cavity of the patient (inside the body) from the outlet 53. After the suture needle 10 have been inserted into the guide tube 52 in a state where the suture needle 10 is being held by the gripping forceps 60, it is preferable to make the suture needle 10 progress without re-holding the suture needle 10 (without releasing the suture needle 10 even for temporally). Further, it is preferable to hold the suture needle 10 such that the distal end 11 of the needle body 1 does not exit first upon when the suture needle 10 exits into the abdominal cavity of the patient from the outlet 53. That is, it is preferable to hold the suture needle 10 such that trunk 13 or the base end 12 of the needle body 1 exits first into the abdominal cavity. When the suture needle 10 is delivered out to the inside of the abdomen P of the patient (inside the body), the needle body 1 returns to its original arc shape as shown in FIG. 9. That is, the inside of the abdomen P of the patient (inside the body) is at the predetermined temperature or higher, so the needle body 1 is restored to the original arc shape. After the needle body 1 has been restored to the original arc shape, the patient's body is sutured by the suture needle 10. For example, an organ such as stomach or bowel is sutured. After the suture, the needle body 1 of the suture needle 10 is cooled within the patient's body to make it deformable, and then the suture needle 10 is pulled out from the patient's body through the guide tube 52. Since the needle body 1 is cooled, it can be deformed so as to easily pass through the guide tube 52. Due to this, the suture needle 10 can smoothly be pulled out to the outside of the patient's body. Even in a case where cooling cannot be performed and the Young's modulus of the needle body 1 does not decrease, the needle body 1 is in the super-elastic state at the temperature of Af point or higher, and thus it can be deformed by being pulled strongly with a normal gripping device and can be taken out through the guide tube 52.

Further, when the needle body 1 is deformed outside the patient's body, it is preferable to bend the needle body 1 to be in a shape in which the distal end 11 of the needle body 1 is curled inward. By so doing, the distal end 11 of the needle body 1 will not be caught by the inside of the guide tube 52 and within the patient's body. Further, it is preferable to maintain the state where the suture needle 10 is held by the gripping forceps 60 after the suture needle 10 has been inserted into the patient's body. The suture can be performed in the state where the suture needle 10 is held by the gripping forceps 60. Due to this, the suture can easily be performed.

As is apparent from the foregoing description, in the suture needle 10 above, the needle body 1 is constituted of the shape memory alloy and has the arc shape as its original shape. Further, when the needle body 1 reaches the predetermined temperature or higher, it is restored to the arc shape. According to this configuration, the shape of the needle body 1 can freely be changed outside the patient's body before the suture needle 10 is inserted into the patient's body. That is, since the outside of the patient's body is at the predetermined temperature or lower, the needle body 1 can be deformed by the characteristic of the shape memory alloy. Due to this, the suture needle 10 can quickly and safely be introduced into the patient's body. For example, in the above embodiment, the needle body 1 is deformed to the ring shape outside the patient's body, so the distal end 11 and the base end 12 of the needle body 1 are not caught by a wall surface of the guide tube 52 and the like when the suture needle 10 is introduced into the patient's body through the guide tube 52 of the trocar 50. Further, even when the suture needle 10 has a shape with a larger diameter than that of the guide tube 52 of the trocar 50, it can be inserted to the inside of the patient's body by deforming the needle body 1 to be in a shape that can be inserted into the guide tube 52. Meanwhile, once the suture needle 10 is introduced into the patient's body, the needle body 1 returns to its original arc shape. That is, since the inside of the patient's body is at the predetermined temperature or higher, the needle body 1 constituted of the shape memory alloy is restored to the original arc shape by the characteristic thereof. When the needle body 1 is restored to the arc shape, it returns to the shape by which the patient's body can be sutured, so operability is improved and the suture in the surgery can be performed promptly. In the above embodiment, the arc-shaped needle body 1 can suture the organ such as the stomach or bowel of the patient. As described above, in the suture needle 10 above, the needle body 1 is capable of being deformed outside the patient's body upon the surgery and of being restored to the original shape within the patient's body, thus the surgery can be performed safely and promptly. The suture needle 10 above is suitable especially for endoscopic surgeries in which a surgical instrument is inserted to the patient's body through a small incision.

Further, according to the above configuration, the connector 2 connecting the needle body 1 and the suture thread 90 is constituted of austenitic stainless steel. Due to this, a shape of the portion connecting the suture needle 10 and the suture thread 90 can be suppressed from changing between outside and inside the patient's body. Due to this, decrease in the connecting force between the suture needle 10 and the suture thread 90 is suppressed, and thus they can be connected firmly.

Further, according to the above configuration, since the needle body 1 and the suture thread 90 are connected in the state where the projection 14 of the needle body 1 and the distal end 91 of the suture thread 90 are inserted in the insert hole 21 of the connector 2, they can be connected firmly. Further, according to the above configuration, since the tapered portion 16 of the needle body 1 and the chamfered portion 26 of the connector 2 face each other, stress at the portion where the needle body 1 and the connector 2 make contact is dispersed and the needle body 1 becomes less likely to break. Further, according to the above configuration, since the glossiness of the needle body 1 and the connector 2 is suppressed, the needle body 1 and the connector 2 do not excessively reflect light of the endoscope when the suture needle 10 is observed by the endoscope. Due to this, the suture needle 10 can more easily be observed. Further, burden on an observer's eyes can be reduced.

Further, since the needle body 1 is constituted, for example, of Ti-Ni-based shape memory alloy, it is configured to be in a parent phase (austenite phase) at the body temperature. This alloy is in the austenite phase at the temperature of the transformation point Af point or higher, but it has the super-elasticity by which the needle body 1 becomes easily deformable due to stress-induced martensitic transformation (pseudoelasticity) when a deforming force of a certain level or higher is applied to the needle body 1. Thus, the suture needle 10 can pass through the guide tube 52 by being strongly pulled after the suture because the needle body 1 is deformed, although a larger force is needed in such a case than the case where the suture needle 10 passes through the guide tube 52 by cooling the suture needle 10.

Although it had been impossible to bring forth such deformation in a conventional needle constituted of SUS, the needle body 1 above is deformable, and thus, even if the suture thread 90 is cut after the suture and the suture needle 10 is left inside the body, forceps that can enter the guide tube 52 can be used to hold the suture needle 10 and pull it out to the outside of the body.

As above, an embodiment has been described, however, specific configurations are not limited to the above embodiment. For example, the above embodiment described the laparoscopic surgery as an example of the surgery using the suture needle 10, however, no limitation is made hereto, and the suture needle 10 can be used in other surgeries. For example, the suture needle 10 may be used in other endoscopic surgeries such as a thoracoscopic surgery and an arthroscopic surgery.

Further, the distal end 11 of the needle body 1 is sharply pointed in the above embodiment, however, no limitation is made hereto. For example, in another embodiment, the distal end 11 of the suture needle 10 may have a spherical shape.

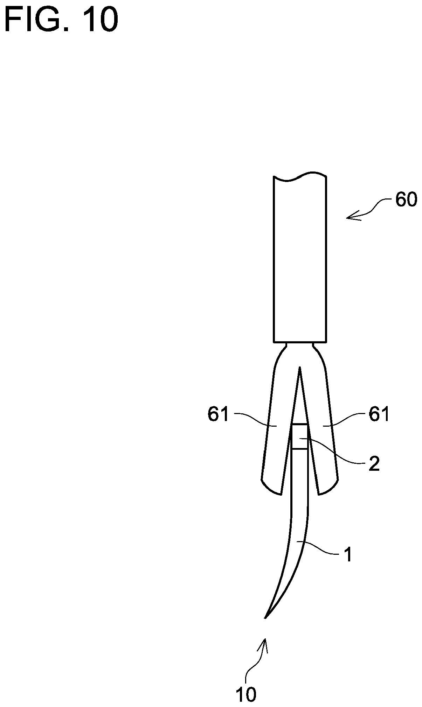

Further, in the above embodiment, the suture needle 10 is deformed in the ring shape outside the patient's body, however, a deformed shape is not particularly limited. For example, as shown in FIG. 10, the needle body 1 may be deformed to extend substantially in a linear shape. Even with such a configuration, the suture needle 10 is safely introduced into the patient's body without the deformed needle body 1 being caught on the wall surface of the guide tube 52 of the trocar 50.

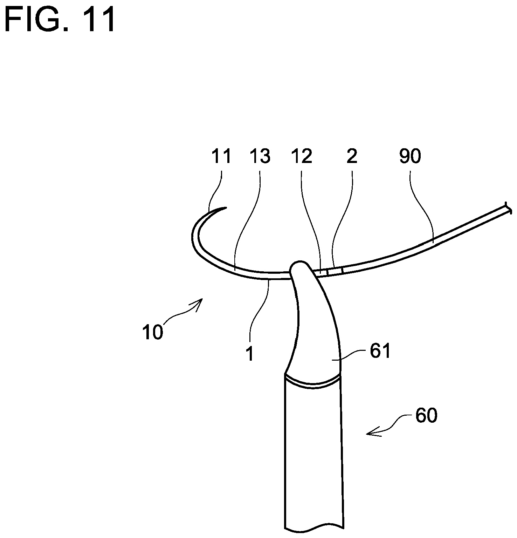

Further, when the gripping forceps 60 hold the suture needle 10, as shown in FIG. 11, it is preferable for the suture needle 10 to be held such that the grip portions 61 of the gripping forceps 60 form substantially a right angle relative to the base end 12 of the needle body 1.

Further, in the above embodiment, the needle body 1 and the connector 2 are connected by crimp fixation, however, no limitation is made to this configuration. In another embodiment, the needle body 1 and the connector 2 may be connected by welding, thread engagement, adhesion, or brazing.

Specific examples of the present invention have been described in detail, however, these are mere exemplary indications and thus do not limit the scope of the claims. The art described in the claims include modifications and variations of the specific examples presented above. Technical features described in the description and the drawings may technically be useful alone or in various combinations, and are not limited to the combinations as originally claimed. Further, the art described in the description and the drawings may concurrently achieve a plurality of aims, and technical significance thereof resides in achieving any one of such aims.

### Reference Signs List

- 1:: Needle body
- 2:: Connector
- 10:: Suture needle
- 11:: Distal end
- 12:: Base end
- 13:: Trunk
- 14:: Projection
- 16:: Tapered portion
- 21:: Insert hole
- 26:: Chamfered portion
- 50:: Trocar
- 51:: Inlet
- 52:: Guide tube
- 53:: Outlet
- 60:: Gripping forceps
- 61:: Grip portions
- 62:: Link mechanism
- 63:: Rod
- 64:: Tube
- 65:: Handle
- 66:: Grip surfaces
- 90:: Suture thread
- 91:: Distal end
- 211:: Distal end portion
- 212:: Proximal end portion
- M:: Patient
- P:: Abdomen

## Claims

1. A suture needle for endoscopic surgery, the suture needle comprising:
a needle body;
a suture thread; and
a connector configured to connect the needle body and the suture thread,
wherein
the needle body is constituted of a shape memory alloy, having an arc shape as its original shape, and is configured to be restored to the arc shape at a predetermined temperature or higher, and
the connector is constituted of a material different from the shape memory alloy.

2. The suture needle according to claim 1, wherein
the connector is constituted of austenitic stainless steel.

3. The suture needle according to claim 1 or 2, wherein
the needle body comprises a base end and a projection projecting from the base end,
an insert hole is provided in the connector, and
the projection is inserted in one end portion of the insert hole and the suture thread is inserted in another end portion of the insert hole.

4. The suture needle according to any one of claims 1 to 3, wherein
an end portion of the projection on a base end side of the needle body is tapered,
one end portion of the connector is chamfered, and
the tapered end portion and the chamfered one end portion face each other.
